Europäisches Patentamt

European Patent Office

Office européen des brevets

(19)

(11) Veröffentlichungsnummer: **0 233 571**
**A2**

(12) **EUROPÄISCHE PATENTANMELDUNG**

(21) Anmeldenummer: **87101741.4**

(22) Anmeldetag: **09.02.87**

(51) Int. Cl.⁴: **C07D 213/30**

(30) Priorität: **15.02.86 DE 3604873**

(43) Veröffentlichungstag der Anmeldung:
**26.08.87 Patentblatt 87/35**

(84) Benannte Vertragsstaaten:
**CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Luksza, Michael Dr.**
**Gaustrasse 54 A**
**D-6702 Bad Duerkheim(DE)**

(54) **Verfahren zur Herstellung von Pyridinolcarbamat.**

(57) Es wird ein Verfahren zur Herstellung von Pyridinolcarbamat beschrieben, das dadurch gekennzeichnet ist, daß man einen Carbaminsäureester der Formel

$$H_3C\text{-}NH\text{-}CO\text{-}O\text{-}R \quad ,$$

worin R für einen Methyl-, Ethyl-, n-Propyl-, i-Propyl-
, n-Butyl-, i-Butyl-oder t-Butylrest steht, in flüssiger
Phase bei erhöhter Temperatur mit 2,6-Bis-
(hydroxymethyl)pyridin in Gegenwart eines Umesterungskatalysators vom Lewis-Säurentyp umsetzt.

EP 0 233 571 A2

## Verfahren zur Herstellung von Pyridinolcarbamat

Die Erfindung betrifft ein Verfahren zur Herstellung von Pyridinolcarbamat (= 2,6-Bis-(hydroxymethyl)pyridin-2,6-bis(N-methylcarbamat)).

Pyridinolcarbamat ist ein Bradykininantagonist mit antiarteriosklerotischer Wirkung (vgl. Ehrhart und Ruschig; Arzneimittel II, S. 435, Verlag Chemie Weinheim 1972) und ist durch Umsetzung von 2,6-Bis(hydroxymethyl)pyridin mit Methylisocyanat zugänglich. Aus der FR-A-1 396 624 ist die Umsetzung dieser beiden Substanzen in Pyridin bekannt. Nach einer Reaktionszeit von insgesamt 15 h (12 h bei Raumtemperatur, 3 h in der Siedehitze) erhält man das gewünschte Produkt in nahezu quantitativer Ausbeute. Nach T. Szén und A. Szöllosy (Arch. Pharm 310, 759-762, 1977), entstehen als Nebenprodukte (2 bis 5 %) durch Reaktion des Pyridinolcarbamats mit überschüssigem Methylisocyanat die Mono-und Diallophanate, bzw. als Abbauprodukt das Trismethylisocyanurat.

Wesentlicher Nachteil dieses Verfahrens ist die Verwendung von Methylisocyanat, dessen Herstellung aus Phosgen und Methylamin sowie Handhabung aufgrund der hohen Toxizitäten erhebliche Risiken birgt (vgl. Nachr. Chem. Techn. Lab. 33, - (1985), Nr. 7, 590).

In der ES-A-417 812 ist die Umesterung von 2,6-Bis(hydroxymethyl)pyridin mit Carbaminsäureethylester ($H_3CNHCOOC_2H_5$) in Gegenwart von Al(Oi-C₃H₇)₃ in Xylol als Lösungsmittel zum Pyridinolcarbamat beschrieben. Nach eigenen Untersuchungen konnte das gewünschte Produkt unter den in der Patentschrift genannten Bedingungen (vgl. Zeile 25 bis 30, S. 2; Zeile 1 bis 5, S. 3) nur in Spuren erhalten werden.

Es wurde nun ein Verfahren gefunden, mit dessen Hilfe Pyridinolcarbamat in guter Ausbeute aus einfach zu handhabenden bzw. wenig toxischen Ausgangsverbindungen hergestellt werden kann.

Gegenstand der Erfindung ist ein Verfahren zur Herstellung von Pyridinolcarbamat, das dadurch gekennzeichnet ist, daß man einen Carbaminsäureester der Formel

$H_3C-NH-CO-O-R$ ,

worin R für einen Methyl-, Ethyl-, n-Propyl-, i-Propyl-, n-Butyl-, i-Butyl-oder t-Butylrest steht, in flüssiger Phase bei erhöhter Temperatur mit 2,6-Bis(hydroxymethyl)pyridin in Gegenwart eines Umesterungskatalysators vom Lewis-Säurentyp umsetzt, wobei man die Umesterung in Abwesenheit eines Lösungsmittel oder in einem inerten Lösungsmittel, ausgenommen Xylol, durchführt.

Nach einer bevorzugten Ausführungsform führt man die Umesterung in einem Temperaturbereich zwischen 130 und 180°C, insbesondere zwischen 150 und 170°C durch.

Das erfindungsgemäße Verfahren wird in Gegenwart eines Lewis-sauren Umesterungskatalysators durchgeführt. Beispielsweise sind zu nennen Aluminiumalkoholate, Zinntetrachlorid, Organo-Zinn-Verbindungen, Titanalkoholate, Zirkonalkoholate sowie saure Zeolithe. Die Verwendung von Zinntetrachlorid, Aluminiumtriisopropylat und saurem Zeolith ist bevorzugt. Dabei werden Zinntetrachlorid und Aluminiumtriisopropylat in einer Menge von vorzugsweise 1,0 bis 10 Mol% und saure Zeolithe in einer Menge von vorzugsweise 3 bis 6 Gewichtsprozent eingesetzt.

Zweckmäßig ist es, bei einem Druck von 0,02 bis 1 bar zu arbeiten. Bevorzugt ist atmosphärischer Druck.

Das Molverhältnis der beiden Reaktanden 2,6-Bis(hydroxymethyl)pyridin:Carbaminsäureester beträgt vorzugsweise 1 : 2,5 bis 1 : 8. Bevorzugt arbeitet man mit einem Molverhältnis 2,6-Bis-(hydroxymethyl)pyridin:Carbaminsäureester von 1 : 2,5 bis 1 : 4,0.

Das erfindungsgemäße Verfahren kann lösungsmittelfrei oder vorzugsweise in Gegenwart eines inerten Lösungsmittels, ausgenommen Xylol, durchgeführt werden. Als Lösungsmittel kann man besonders bevorzugt überschüssigen Carbaminsäureester oder 1,2-Dichlorbenzol verwenden. Aber auch andere inerte organische, aliphatische oder aromatische Lösungsmittel mit einem zwischen 150°C und 250°C liegenden Siedepunkt sind geeignet, lediglich das Xylol führt nicht zum gewünschten Ergebnis. Die Ursache hierfür ist nicht bekannt. Als Beispiele für weitere, geeignete Lösungsmittel kann man nennen:

n-Nonan, Decahydronaphthalin, n-Butylcyclohexan, n-Undecan, n-Dodecan, Isopropylbenzol, 1,3-Diethylbenzol, Inden, Tetralin, Anisol, Phenyl-n-propylketon, Benzophenon, Acetophenon, N-Methylformamid, N,N-Dimethylformamid, N,N-Dimethylanilin, N,N-Dimethyl-2-methylanilin, 4-Methoxytoluol, Hexamethylphosphorsäuretriamid, Dimethylsulfoxid, 2-Ethylpyridin, Chinolin, Nitrocyclohexan, Nitrobenzol, 2-Nitrotoluol, 2,4-Dimethyl-1-nitrobenzol, o-Toluolnitril, p-Toluolnitril, Phenylacetonitril, Diethylenglykoldimethylether, Dimethylpropylenharnstoff, Sulfolan.

Das erfindungsgemäße Verfahren wird zweckmäßig so durchgeführt, daß man ein Gemisch der Reaktionspartner im genannten Molverhältnis auf erhöhte Temperatur erhitzt. Über einen, auf eine um ca. 5°C höhere Temperatur als die Siedetemperatur des abzuspaltenden Alkohols thermostatisierten Kühler, wird der freiwerdende Alkohol aus dem Reaktionsgleichgewicht ausgeschleust. Die Umsetzung ist nach 4 bis 12 h abgeschlossen.

Nach dem Abtrennen überschüssigen Carbaminsäureesters, gegebenenfalls des Lösungsmittels und unerwünschter Nebenprodukte, wie Trismethylisocyanurat und Dimethylharnstoff - sie resultieren aus der teilweisen thermischen Zersetzung des Carbaminsäureesters bzw. des Pyridindicarbamates -erhält man das gewünschte Produkt in 40 bis 98 %iger Ausbeute.

Zur weiteren Reinigung empfiehlt es sich, das Pyridinoldicarbamat (I) in Wasser und konzentrierter Salzsäure zu lösen und mit Xylol, Toluol oder Methylenchlorid zu extrahieren. Die wäßrige Phase wird dann mit verdünnter Natriumcarbonatlösung neutralisiert und das Produkt durch Absaugen isoliert.

Die besonderen Vorteile des erfindungsgemäßen Verfahrens liegen in der isocyanatfreien und damit sicherheitstechnisch unproblematischen Synthese des Pyridinoldicarbamates. Die eingesetzten Carbaminsäureester sind zudem auch durch eine neuartige, phosgenfreie Elektrooxidation, ausgehend von Methylformamid, zugänglich - (vgl. deutsche Patentanmeldung P 35 29 531.7). Insgesamt lassen sich sowohl die Carbaminsäureester, die Katalysatorsysteme als auch die Reaktionsführung über ein breiteres Spektrum als bisher variieren.

Nebenprodukte treten nur in geringem Maße auf und sind im wesentlichen auf die Zersetzung des Carbaminsäureesters zurückzuführen. Unumgesetztes 2,6-Bis(hydroxymethyl)pyridin läßt sich extraktiv zurückgewinnen und in die Umsetzung zurückführen.

Die nachfolgenden Beispiele dienen zur weiteren Erläuterung der Erfindung.

Beispiel 1

Eine Lösung von 35 g (0,25 Mol) 2,6-Bis-(hydroxymethyl)pyridin in 178 g (2,0 Mol) N-Methylcarbaminsäuremethylester und 6,5 g (0,025 Mol) Zinntetrachlorid wurde auf 166°C erhitzt, wobei über eine auf 70°C thermostatisierte Kolonnu langsam freigesetztes Methanol abdestillierte. Nach 12 h war die theoretische Menge Alkohol übergegangen. Das Gemisch wurde im Vakuum zur Trockne eingedampft und der Rückstand in heißem Aceton aufgenommen, Unlösliches heiß abfiltriert und anschließend auf 0°C im Eisbad abgekühlt und gefälltes Pyridinolcarbamat abfiltriert. Ausbeute 41 g (65 %).

Beispiel 2

Eine Lösung von 35 g (0,25 Mol) 2,6-Bis-(hydroxymethyl)pyridin in 178 g (2,0 Mol) N-Methylcarbaminsäuremethylester und 2 g saurer Aluminiumzeolith wurden auf 166°C erhitzt, wobei über eine auf 70°C thermostatisierte Kolonnne langsam freigesetztes Methanol abdestillierte. Nach 12 h war die theoretische Menge Alkohol übergegangen. Das Gemisch wurde im Vakuum zur Trockne eingedampft und der Rückstand in heißem Aceton aufgenommen, Unlösliches heiß abfiltriert, anschließend auf 0°C im Eisbad abgekühlt und gefälltes Pyridinolcarbamat abfiltriert. Ausbeute 26 g (41 %).

Beispiel 3

Eine Lösung von 17,5 g (0,125 Mol) 2,6-Bis-(hydroxymethyl)pyridin in 77 g (0,75 Mol) N-Ethylcarbaminsäureethylester und 312 g (0,0125 Mol) Zinntetrachlorid wurde auf 166°C erhitzt, wobei über eine auf 85°C thermostatisierte Kolonnne das freigesetzte Ethanol abdestillierte. Nach 12 h war die theoretische Menge Alkohol übergegangen. Das Gemisch wurde im Vakuum zur Trockne eingedampft und der Rückstand in heißem Aceton aufgenommen. Unlösliches heiß abfiltriert, anschließend auf 0°C im Eisbad abgekühlt und gefälltes Pyridinolcarbamat abfiltriert. Ausbeute 13 g (41 %).

Beispiel 4

Zu einer Lösung von 8,34 g (0,06 Mol) 2,6-Bis(hydroxymethyl)pyridin und 1,22 g (0,006 Mol) Aluminiumtriisopropylat in 40 ml 1,2-Dichlorbenzol tropfte man bei 165°C 13,4 g (0,15 Mol) N-Methylcarbaminsäuremethylester in 10 ml des gleichen Lösungsmittels innerhalb von 2 h zu, wobei über eine auf 70°C thermostatisierte Kolonnne freigesetztes Methanol abdestillierte. Nach 3 h war die theoretische Menge Alkohol übergegangen. Das Gemisch wurde im Vakuum zur Trockne eingedampft und der verbleibende Rückstand in Wasser suspendiert, filtriert und getrocknet. Ausbeute 15,2 g (98 %).

Beispiel 5

Zu einer Lösung von 8,75 g (O,O6 Mol) 2,6-Bis(hydroxymethyl)pyridin und 1,27 g (O,OO6 Mol) Aluminiumtriisopropylat in 5O ml Dichlorbenzol tropfte man bei 165°C 51,5 g (O,5 Mol) N-Methylcarbaminsäureethylester in 5O ml des gleichen Lösungsmittels innerhalb von 2 h zu, wobei über eine auf 85°C thermostatisierte Kolonnne freigesetztes Ethanol abdestillierte. Nach 7 h war die theoretische Menge Alkohol übergegangen. Das Gemisch wurde im Vakuum zur Trockne eingedampft und der verbleibende Rückstand in Wasser suspendiert, filtriert und getrocknet. Ausbeute 13,2 g (87 %).

**Ansprüche**

1. Verfahren zur Herstellung von Pyridinolcarbamat, dadurch gekennzeichnet, daß man einen Carbaminsäureester der Formel

H₃C-NH-CO-O-R ,

worin R für einen Methyl-, Ethyl-, n-Propyl-, i-Propyl-, n-Butyl-, i-Butyl-oder t-Butylrest steht, in flüssiger Phase bei erhöhter Temperatur mit 2,6-Bis(hydroxymethyl)pyridin in Gegenwart eines Umesterungskatalysators vom Lewis-Säurentyp umsetzt, wobei man die Umesterung in Abwesenheit eines Lösungsmittels oder in einem inerten Lösungsmittel, ausgenommen Xylol, durchführt.

2. Verfahren nach Anspruch 1, dadurch gekennzeichnet, daß man als Umesterungskatalysator Zinntetrachlorid, Aluminiumtriisopropylat oder einen sauren Zeolithen einsetzt.

3. Verfahren nach Anspruch 1 oder 2, dadurch gekennzeichnet, daß man die Umesterung in einem Temperaturbereich zwischen 13O und 18O°C durchführt.

4. Verfahren nach einem der Ansprüche 1 bis 3, dadurch gekennzeichnet, daß man bei einem Druck zwischen O,O2 und 1 bar arbeitet.

5. Verfahren nach einem der Ansprüche 1 bis 4, dadurch gekennzeichnet, daß man die Umsetzung in überschüssigem Carbaminsäureester als Lösungsmittel durchführt.

6. Verfahren nach einem der Ansprüche 1 bis 4, dadurch gekennzeichnet, daß man als Lösungsmittel 1,2-Dichlorbenzol verwendet.

7. Verfahren nach einem der Ansprüche 1 bis 6, dadurch gekennzeichnet, daß man ein molares Verhältnis von 2,6-Bis(hydroxymethyl)pyridin:Carbaminsäureester von 1 : 2,5 bis 1 : 8 wählt.